# EUROPEAN PATENT SPECIFICATION

(11) **EP 3 162 405 B1**
(45) Date of publication and mention of the grant of the patent: **17.10.2018**
(21) Application number: 16178436.8
(22) Date of filing: 07.07.2016
(51) Int. Cl.: A61M 25/00, A61M 25/10

(54) **CATHETER**
KATHETER
CATHÉTER

(30) Priority: 27.10.2015 JP 2015210511
(43) Date of publication of application: 03.05.2017
(73) Proprietor: ASAHI INTECC CO., LTD., Nagoya-shi, Aichi 463-0024 (JP)
(72) Inventor: Shimizu, Hirotomo, Nagoya-shi, Aichi 463-0024 (JP); Ishikawa, Masatomo, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 174 685
- WO-A1-2007/067324
- DE-C1- 3 812 188
- US-A1- 2010 004 593

## Description

### TECHNICAL FIELD

The present invention relates to a catheter to be inserted into a tubular organ in human body such as a blood vessel, a gastrointestinal tract and a ureter as well as into a body tissue.

### BACKGROUND ART

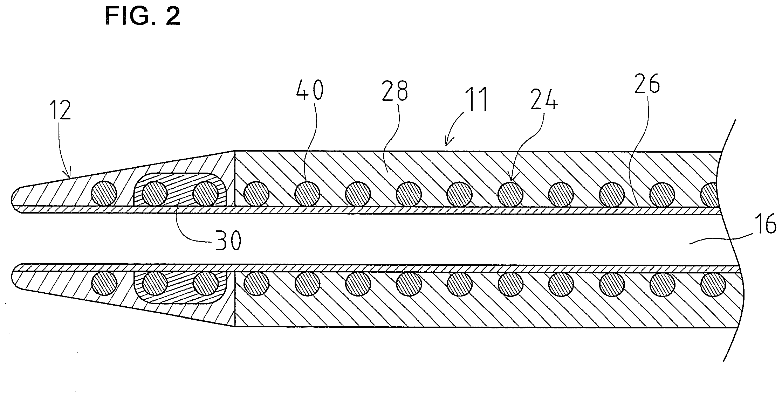

As a catheter to be inserted into a tubular organ in human body such as a blood vessel, a gastrointestinal tract and a ureter as well as into a body tissue, known is a catheter having a braid thereinside, the braid comprising two or more metal element wires. For example, JP 2014-188337 A describes a catheter 100 comprising a tubular body 10, a front end tip 58 attached to the front end of the tubular body 10 and a reinforcement wire 32 arranged inside the tubular body 10 and the front end tip 58 (see Fig. 2 and the like).

In this type of a catheter, the stiffness of the catheter itself is enhanced because a braid is arranged inside the tubular body 10 (hereinafter referred to as the "catheter body") and the front end tip 58, which can prevent the inner cavity of the catheter from collapsing. Further, in a case where the braid in place is replaced which a coil body, the torque transmissibility from the base end of the catheter to the front end will be increased.

US 2010/004593 A1 and EP 2174685 A1 each discloses a balloon catheter comprising a catheter body having a lumen, a resin front end tip having a lumen communicating with the lumen of the catheter body and attached to a front end of the catheter body, a coil body arranged at the catheter body and the front end tip along the lumen, and a reinforcement body covering the coil body.

### SUMMARY OF THE INVENTION

### TECHNICAL PROBLEM

However, in the case of the conventional catheter, even though a braid is arranged inside the catheter body and the front end tip, a front end tip may be detached from a catheter body during procedures and left inside the patient's body.

The present invention is made in view of the above circumstances. An objective of the present invention is to provide a catheter in which the remaining of a front end tip alone in the patient's body may be prevented even in a case where the front end tip is detached from a catheter body.

### SOLUTION TO PROBLEM

In order to achieve the above objective, a first aspect of the present invention is a catheter comprising a catheter body having a lumen and a resin front end tip having a lumen communicating with the lumen of the above catheter body and attached to the front end of the above catheter body and a coil body and/or a braid arranged at the above catheter body and the above front end tip along the above lumens, wherein a reinforcement body covering the above coil body and/or the above braid is provided at the above front end tip and the reinforcement body has a protrusion part extending toward the outer periphery of the above front end tip.

Further, in a second aspect of the present invention, the above reinforcement body comprises a resin harder than a resin constituting the above front end tip.

Further, in a third aspect of the present invention, the above reinforcement body is arranged in such a way to span the above catheter body and the above front end tip (i.e., the above reinforcement body is arranged in such a way to extend from the above catheter body to the above front end tip).

Further, in a fourth aspect of the present invention, the above reinforcement body is integrally formed with the above catheter body. Further, in a fifth aspect of the present invention, the reinforcement body is provided as a separate body from the catheter body.

Furthermore, in a sixth aspect of the present invention, the front end tip has a tapered outer periphery in which the diameter is gradually decreased toward a forefront part of the catheter.

### ADVANTAGEOUS EFFECT OF THE INVENTION

According to the first aspect of the present invention, a catheter comprises a catheter body having a lumen, a resin front end tip having a lumen communicating with the lumen of the above catheter body and attached to the front end of the catheter body, and a coil body and/or a braid arranged at the catheter body and the front end tip along the lumens, a reinforcement body covering the coil body and/or the braid being provided at the front end tip. Therefore, the remaining of the front end tip alone in the patient's body can be prevented even in a case where the front end tip is detached from the catheter body. In addition, as the reinforcement body has a protrusion part extending toward the outer periphery of the front end tip, the remaining of the front end tip alone in the patient's body can further be prevented even in a case where the front end tip is detached from the catheter body.

Further, according to the second aspect of the present invention, the reinforcement body comprises a resin harder than a resin constituting the front end tip. As a result, in addition to the effect of the first aspect of the present invention, the remaining of the front end tip alone in the patient's body can further be prevented even in a case where the front end tip is detached from the catheter body.

Further, according to the third aspect of the present invention, the reinforcement body is arranged in such a way to span the catheter body and the front end tip. As a result, in addition to the effect of the first or second aspect of the present invention, the detachment of the front end tip from the catheter body itself can be prevented, which, in turn, can prevent the front end tip alone from remaining in the patient's body.

Further, according to the fourth aspect of the present invention, the reinforcement body is integrally formed with the catheter body. As a result, in addition to the effect of any one of the first aspect of the present invention to the third aspect of the present invention, the detachment of the front end tip from the catheter body can further be prevented, which, in turn, can prevent the front end tip alone from remaining in the patient's body.

### BRIEF DESCRIPTION OF THE DRAWINGS

Fig. 1 shows a front view illustrating an embodiment of the catheter according to the present invention.
Fig. 2 shows a partial cross sectional view of an exemplary catheter not forming part of the present invention (reference).
Fig. 3 shows a partial cross sectional view of the catheter according to the first embodiment of the present invention.
Fig. 4 shows a partial cross sectional view of the catheter according to the second embodiment of the present invention.
Fig. 5 shows a partial cross sectional view of the catheter according to the second alternative embodiment of the present invention.
Fig. 6 shows a partial cross sectional view of the catheter according to the third embodiment of the present invention.
Fig. 7 shows a partial cross sectional view of the catheter according to the third alternative embodiment of the present invention.

### DESCRIPTION OF EMBODIMENTS

Below, the present invention will be described with reference to the drawings.

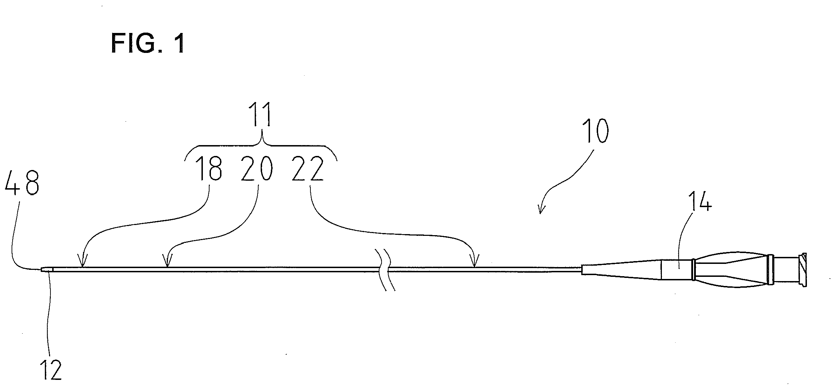

Fig. 1 shows a front view illustrating an embodiment of the catheter according to the present invention, and Fig. 2 shows an exemplary catheter.

In Fig. 1, a catheter 10 comprises a catheter body 11, a flexible front end tip 12 attached to the front end of the above catheter body 11 and a connector 14 attached to the base end of the catheter body 11.

More specifically, the catheter body 11 comprises a hollow elongated member having a lumen 16 into which a guide wire will be inserted. In this example, the full length of the catheter body 11 is about 1500 mm. A portion for about 200 mm from the front end of the catheter body 11 corresponds to a front end part 18 having an outer diameter of about 0.88 mm (0.85 to 0.90 mm), and a portion for about 600 mm from the base end of the front end part 18 corresponds to an intermediate part 20 having an outer diameter slightly larger than that of the front end part 18. Further, a portion from the base end of the intermediate part 20 to the connector 14 corresponds to a base end part 22 having an outer diameter even larger than that of intermediate part 20.

Moreover, as shown in Fig. 2, the catheter body 11 has a hollow coil body 24, an inner resin layer 26 covering the inner periphery of the coil body 24 and an outer resin layer 28 covering the outer periphery of the coil body 24.

The inner resin layer 26 has a tube-like shape extending throughout the full length at the innermost part of the catheter body 11. In addition, the lumen 16 of the catheter body 11 is formed in the inner cavity of the inner resin layer 26. Moreover, the inner resin layer 26 axially extends from the front end of the catheter body 11 to the side of the front end.

Note that there is no particular limitation for resin materials for forming the aforementioned inner resin layer 26, but resin materials conferring flexibility and moderate plasticity will appropriately be selected. Among these, for example, polytetrafluoroethylene (PTFE), which has excellent lubricity, is preferably used in view of the slideability of a guide wire.

The coil body 24 is a hollow twisted wire coil in which two or more metal element wires 40 each having a circular cross section are twisted (10 element wires in this embodiment). Further, since the coil body 24 is heat-treated by the known method after the two or more metal element wires 40 are twisted, the residual stress of the coil body 24 is removed.

There is no particular limitation for a metal material for forming the metal element wires 40 of the coil body 24. For example, a metal material such as stainless steel and a superelastic alloy such as a Ni-Ti alloy may each independently be used to form the coil body 24, or they may be used in combination to form the coil body.

The outer resin layer 28, which constitutes the outermost layer of the catheter body 11, extends throughout the full length of the catheter body 11 in a state where the outer periphery of the coil body 24 is covered such that the outer periphery of the metal element wires 40 which constitutes the coil body 24 is not be exposed outside from the outer surface of the catheter body 11.

Further, in the outer resin layer 28, the thickness of a portion corresponding to the outermost layer of the intermediate part 20 of the catheter body 11 is larger than that of a portion corresponding to the outermost layer of the front end part 18 of the catheter body 11. Moreover, the thickness of a portion corresponding to the outermost layer of the base end part 22 of the catheter body 11 is larger than that of a portion corresponding to the outermost layer of the intermediate part 20 of the catheter body 11.

Further, the hardness of the outer resin layer 28 at the front end part 18, the intermediate part 20 and the base end part 22 of the catheter body 11 is increased from the front end part 18 to the base end part 22 in incremental steps. Thereby, the plasticity of the base end part 22, the intermediate part 20 and the front end part 18 of the catheter body 11 is increased from the base end part 22 to the front end part 18 in incremental steps.

There is no particular limitation for a resin material for forming the outer resin layer 28, but a resin material conferring flexibility and moderate plasticity may appropriately be selected. Materials for forming the aforementioned outer resin layer 28 can include polyamide elastomers and the like.

Meanwhile, the front end tip 12 provided at the front end of the catheter body 11 has an elongated tubular overall shape, and comprises a lumen communicating with the lumen 16 of the catheter body 11, and has a plasticity well higher than that of the catheter body 11. In addition, the above front end tip 12 has a tapered outer periphery in which the diameter is gradually decreased toward a forefront part 48.

There is also no particular limitation for a resin material forming the front end tip 12, but a resin material having higher plasticity than that for forming the inner resin layer 26 and the outer resin layer 28 may suitably be used. Examples of such a resin material can include polyurethane elastomers. Note that in this example, tungsten powder is mixed into the front end tip 12. Thereby, the visibility of the front end tip 12 under radioscopy is enhanced advantageously.

Moreover, the inner periphery of the front end tip 12 is formed with the inner resin layer 26 extending from the front end of the catheter body 11, and the coil body 24 which continues from the front end of the catheter body 11 is wound and embedded around the outer periphery of the inner resin layer 26 in the inside of the front end tip 12.

Moreover, in order to prevent the detachment of the front end tip 12 from the coil body 24, a reinforcement body 30 comprising a resin harder than a resin material of the front end tip 12 is fixed to the front end tip 12 and the coil body 24 in the inside of the front end tip 12. Thereby, the reinforcement body 30 fixed to the front end tip 12 and the coil body 24 can prevent the detachment of the front end tip 12 from the coil body 24 even in a case where the front end tip 12 is detached from the catheter body 11, which in turn, can prevent the detachment of the front end tip 12 from the catheter body 11.

Note that, in this embodiment, the reinforcement body 30 is provided as a separate body from the catheter body 11. Further, in this example, the reinforcement body 30 does not have an after-mentioned protrusion part extending toward the outer periphery of the front end tip.

Examples of a material for the reinforcement body 30 can include polyamide elastomers. In this embodiment, a polyamide elastomer harder than the outer resin layer 28 is used.

### (First embodiment)

Next, the first embodiment of the present invention will be described. The catheter according to the first embodiment represents an embodiment in which the coil body 24 of the catheter shown in Fig.2 is replaced with a braid body 124. Further, since the catheter according to the first embodiment has the same appearance as the catheter 10 described above, only the internal structure is described with reference to a drawing with an overall view omitted.

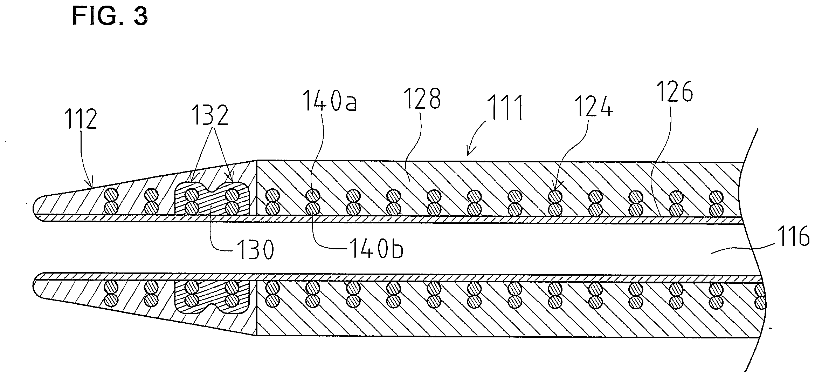

Fig. 3 shows a partial cross sectional view of the catheter according to the first embodiment of the present invention. In Fig. 3, a catheter body 111 comprises a hollow elongated member having a lumen 116 into which a guide wire will be inserted. As shown in Fig. 3, the catheter body 111 has a braid body 124, an inner resin layer 126 covering the inner periphery of the braid body 124 and an outer resin layer 128 covering the outer periphery of the braid body 124.

The inner resin layer 126 has a tube-like shape extending throughout the full length at the innermost part of the catheter body 111. Moreover, the inner resin layer 126 axially protrudes from the front end of the catheter body 111 to the side of the front end. Note that for a resin material for forming the aforementioned inner resin layer 126, a similar material to the inner resin layer 26 in the first embodiment may be used.

The braid body 124 has a structure in which two metal element wires 140a and 140b are woven each other in such a way to form a mesh-like structure. In addition, the braid body 124 having the aforementioned mesh-like structure is wound around the outer periphery of the inner resin layer 126 throughout the full length.

Further, as in the inner resin layer 126, the braid body 124 is wound around the outer periphery of the inner resin layer 126 which axially extends from the front end of the catheter body 111 to the side of the front end, and protruded from the front end of the catheter body 111.

There is no particular limitation for the type of a metal material for the metal element wires 140a and 140b of the braid body 124, but, for example, a wire material comprising tungsten, stainless steel and the like may suitably be used. In addition, a tungsten wire material, which has good visibility under radioscopy, is more suitably used.

Note that there is also no particular limitation for the size of the metal element wires 140a and 140b, but it may appropriately selected depending on the sizes (outer diameters) of the catheter body 111 and the front end tip 112 and the like. According to this embodiment, the diameters of the metal element wires 140a and 140b are about 0.023 mm. In addition, one metal element wire 140a and one metal element wire 140b are woven each other to form the braid body 124.

The outer resin layer 128 constitutes the outermost layer of the catheter body 111. In addition, it extends throughout the full length of the catheter body 111 in a state where the braid body 124 is covered such that the metal element wires 140a and 140b constituting the braid body 124 are not exposed outside from the outer surface of the catheter body 111.

Note that for a resin material for forming the outer resin layer 128, a similar material to the outer resin layer 28 in the first embodiment may be used.

Further, the front end tip 112 provided at the front end of the catheter body 111 has an elongated tubular overall shape, and comprises a lumen communicating with the lumen 116 of the catheter body 111, and has a plasticity well higher than that of the catheter body 111. In addition, the front end tip 112 has a tapered outer periphery in which the diameter is gradually decreased toward a forefront part 48.

Note that for a resin material for forming the front end tip 112, a similar material to the front end tip 12 in the first embodiment may be used.

The inner periphery of the front end tip 112 is formed with the inner resin layer 126 extending from the front end of the catheter body 111, and the braid body 124 which continues from the front end of the catheter body 111 is wound and embedded around the outer periphery of the inner resin layer 126 in the inside of the front end tip 112.

Further, in order to prevent the detachment of the front end tip 112 from the braid body 124, a reinforcement body 130 comprising a resin harder than a resin material of the front end tip 112 is fixed to the front end tip 112 and the coil body 124 in the inside of the front end tip 112. Thereby, the reinforcement body 130 fixed to the front end tip 112 and the braid body 124 can prevent the detachment from the braid body 124 even in a case where the front end tip 112 is detached from the catheter body 111, which, in turn, can prevent the detachment of the front end tip 112 from the catheter body 111.

Then, the reinforcement body 130 may be any as long as it can be fixed to the front end tip 112 and the coil body 124, but it is preferably formed in such a way to have a protrusion part 132 extending toward the outer periphery in order to effectively prevent the detachment of the front end tip 112 from the catheter body 111 in the axial direction of the catheter.

Note that the protrusion part 132 is provided at two positions in the reinforcement body 130 according to this embodiment, but may be provided at one position or at three or more positions.

### (Second embodiment)

Next, the second embodiment of the present invention will be described. The catheter according to the second embodiment differs form the catheter according to first embodiment in that the position of the reinforcement body. That is, the reinforcement body of the catheter according to the second embodiment is arranged in such a way to span the catheter body and the front end tip while the reinforcement body of the catheter according to the first embodiment is arranged only within the front end tip.

Meanwhile, since the catheter according to the second embodiment also has the same appearance as the catheter 10 according to the first embodiment, only the internal structure is described with reference to a drawing with an overall view omitted.

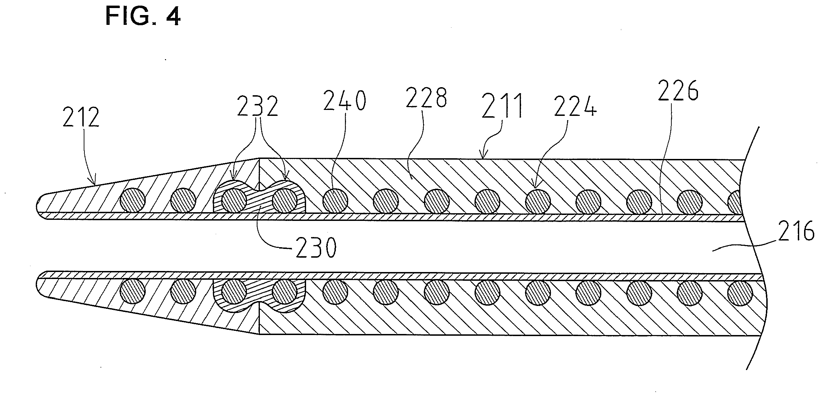

Fig. 4 shows a partial cross sectional view of the catheter according to the second embodiment of the present invention. In Fig. 4, a catheter body 211 comprises a hollow elongated member having a lumen 216 into which a guide wire will be inserted. As shown in Fig. 4, the catheter body 211 has a coil body 224, an inner resin layer 226 covering the inner periphery of the coil body 224 and an outer resin layer 228 covering the outer periphery of the coil body 224.

The inner resin layer 226 has a tube-like shape extending throughout the full length at the innermost part of the catheter body 211. Moreover, the inner resin layer 226 axially protrudes from the front end of the catheter body 211 to the side of the front end. Note that for a resin material for forming the aforementioned inner resin layer 226, a similar material to the inner resin layer 26 in the first embodiment may be used.

The coil body 224 is a hollow twisted wire coil in which two or more metal element wires 240 each having a circular cross section are twisted (10 element wires in this embodiment). Further, since the coil body 224 is heat-treated by the known method after the two or more metal element wires 240 are twisted, the residual stress of the coil body 224 is removed.

The same material as the metal element wires 40 according to the first embodiment can be used for a metal material of the metal element wires 240 of the coil body 224.

The outer resin layer 228 constitutes the outermost layer which forms the outer surface of the catheter body 211. In addition, it extends throughout the full length of the catheter body 211 in a state where the outer periphery of the coil body 224 is covered such that the peripheries of the metal element wires 240 which form the coil body 224 are not exposed outside from the outer surface of the catheter body 211.

Note that for a resin material for forming the outer resin layer 228, a similar material to the outer resin layer 28 in the first embodiment can be used.

Meanwhile, the front end tip 212 provided at the front end of the catheter body 211 has an elongated tubular overall shape, and comprises a lumen communicating with the lumen 216 of the catheter body 211, and has a plasticity well higher than that of the catheter body 211. In addition, the front end tip 212 has a tapered outer periphery in which the diameter is gradually decreased toward a forefront part 48.

Note that for a resin material for forming the front end tip 212, a similar material to the front end tip 12 in the first embodiment may be used.

The inner periphery of the front end tip 212 is formed with the inner resin layer 226 extending from the front end of the catheter body 211, and the coil body 224 which continues from the front end of the catheter body 211 is wound and embedded around the outer periphery of the inner resin layer 226 in the inside of the front end tip 212.

Further, a reinforcement body 230 comprising a resin harder than the resin material of the front end tip 212 which is arranged in such a way to span the catheter body 211 and the front end tip 212 is fixed to the front end part of the catheter body 211 and the base end part of the front end tip 212. Thereby, the detachment of the front end tip 212 from the catheter body 211 can be prevented. Further, the reinforcement body 230 fixed to the front end tip 212 and the coil body 224 can prevent the detachment of the front end tip 212 from the coil body 224 even in a case where the front end tip 212 is detached from the catheter body 211, which, in turn, can prevent the detachment of the front end tip 212 from the catheter body 211.

Then, the reinforcement body 230 may be any as long as it can be fixed to the front end tip 212 and the coil body 224, but it is preferably formed in such a way to have a protrusion part 232 extending toward the outer periphery in order to effectively prevent the detachment of the front end tip 212 from the catheter body 211 in the axial direction of the catheter.

Note that the protrusion part 232 is provided at two positions in the reinforcement body 230 in this embodiment, but may be provided at one position or at three or more positions.

Next, an alternative of the second embodiment of the present invention will be described. The catheter according to this embodiment differs form the catheter according to first embodiment in that the position of the reinforcement body is different. That is, the reinforcement body of the catheter is arranged in such a way to span the catheter body and the front end tip while the reinforcement body of the catheter according to the second embodiment is arranged only within the front end tip. Further, since the catheter also has the same appearance as the catheter 10 according to the first embodiment, only the internal structure is described with reference to a drawing with an overall view omitted.

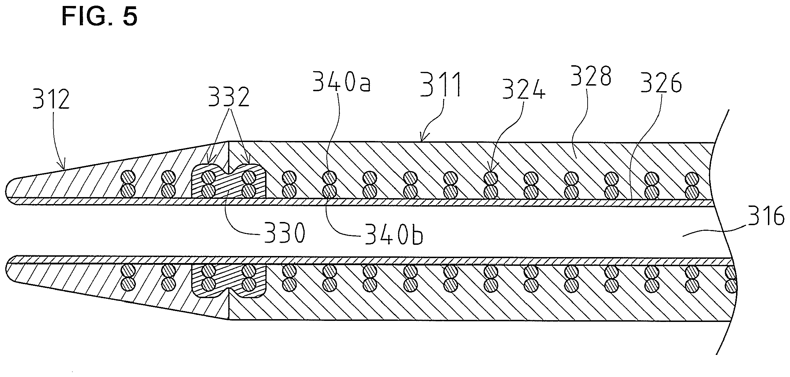

Fig. 5 shows a partial cross sectional view of the catheter according to the second alternative embodiment of the present invention. In Fig. 5, a catheter body 311 comprises a hollow elongated member having a lumen 316 into which a guide wire will be inserted. As shown in Fig. 5, the catheter body 311 has a braid body 324, an inner resin layer 326 covering the inner periphery of the braid body 324 and an outer resin layer 328 covering the outer periphery of the braid body 324.

The inner resin layer 326 has a tube-like shape extending throughout the full length at the innermost part of the catheter body 311. Moreover, the inner resin layer 326 axially protrudes from the front end of the catheter body 311 to the side of the front end. Note that for a resin material for forming the aforementioned inner resin layer 326, a similar material to the inner resin layer 26 in the first embodiment may be used.

The braid body 324 has a structure in which two metal element wires 340a and 340b are woven each other so as to form a mesh-like state. In addition, the braid body 324 having the aforementioned mesh-like structure is wound around the outer periphery of the inner resin layer 326 throughout the full length.

Further, as in the inner resin layer 326, the braid body 324 is wound around the outer periphery of the inner resin layer 326 which axially extends from the front end of the catheter body 311 to the side of the front end, and protruded from the front end of the catheter body 311.

The same material as the metal element wires 140a and 140b of the braid body 124 in the second embodiment may be used for a metal material which constitutes the metal element wires 340a and 340b of the braid body 324.

The outer resin layer 328 comprises the outermost layer of the catheter body 311. In addition, it extends throughout the full length of the catheter body 311 in a state where the braid body 324 is covered such that the metal element wires 340a and 340b constituting the braid body 324 are not exposed outside from the outer surface of the catheter body 311.

Note that for a resin material for forming the outer resin layer 328, a similar material to the outer resin layer 28 in the first embodiment can be used.

Further, the front end tip 312 provided at the front end of the catheter body 311 has an elongated tubular overall shape, and comprises a lumen communicating with the lumen 316 of the catheter body 311, and has a plasticity well higher than that of the catheter body 311. In addition, the above front end tip 312 has a tapered outer periphery in which the diameter is gradually decreased toward a forefront part 48.

Note that for a resin material for forming the front end tip 312, a similar material to the front end tip 12 in the first embodiment may be used.

The inner periphery of the front end tip 312 is formed with the inner resin layer 326 extending from the front end of the catheter body 311, and the braid body 324 which continues from the front end of the catheter body 311 is wound and embedded around the outer periphery of the inner resin layer 326 in the inside of the front end tip 312.

Further, a reinforcement body 330 comprising a resin harder than the resin material of the front end tip 312 which is arranged in such a way to span the catheter body 311 and the front end tip 312 is fixed to the front end part of the catheter body 311 and the base end part of the front end tip 312. Thereby, the detachment of the front end tip 312 from the catheter body 311 can be prevented. Further, the reinforcement body 330 fixed to the front end tip 312 and the coil body 324 can prevent the detachment of the front end tip 312 from the coil body 324 even in a case where the front end tip 312 is detached from the catheter body 311, which, in turn, can prevent the detachment of the front end tip 312 from the catheter body 311.

Here, the reinforcement body 330 may be any as long as it can be fixed to the front end tip 312 and the coil body 324, but it is preferably formed in such a way to have a protrusion part 332 extending toward the outer periphery in order to effectively prevent the detachment of the front end tip 312 from the catheter body 311 in the axial direction of the catheter.

Note that the protrusion part 332 is provided at two positions in the reinforcement body 330 in this embodiment, but may be provided at one position or at three or more positions.

### (Third embodiment)

Next, the third embodiment of the present invention will be described. The catheter according to the third embodiment differs form the catheters according to the first and the second embodiment in that the configuration of the reinforcement body is different. That is, the reinforcement body of the catheter according to the third embodiment is integrally formed with the catheter body while the reinforcement bodies of the catheters according to the other embodiments are provided as separate bodies from the catheter bodies.

Note that since the catheter according to the third embodiment has the same appearance as the catheter 10 according to the first embodiment, only the internal structure is described with reference to a drawing with an overall view omitted.

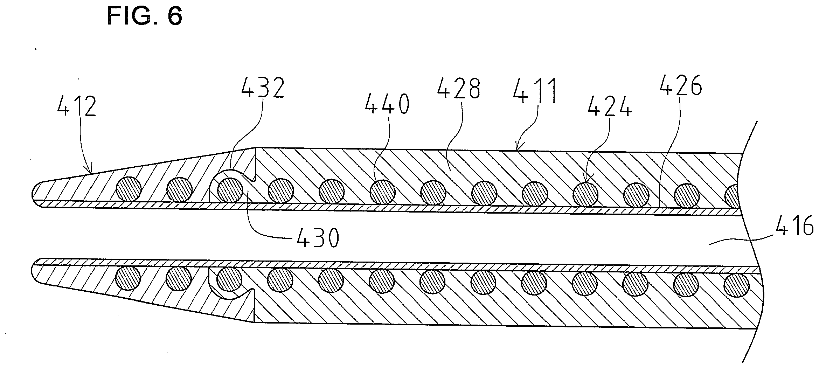

Fig. 6 shows a partial cross sectional view of the catheter according to the third embodiment of the present invention. In Fig. 6, a catheter body 411 comprises a hollow elongated member having a lumen 416 into which a guide wire will be inserted. As shown in Fig. 6, the catheter body 411 has a coil body 424, an inner resin layer 426 covering the inner periphery of the coil body 424 and an outer resin layer 428 covering the outer periphery of the coil body 424.

The inner resin layer 426 has a tube-like shape extending throughout the full length at the innermost part of the catheter body 411. Moreover, the inner resin layer 426 axially protrudes from the front end of the catheter body 411 to the side of the front end. Note that for a resin material for forming the aforementioned inner resin layer 426, a similar material to the inner resin layer 26 in the first embodiment may be used.

The coil body 424 is a hollow twisted wire coil in which two or more metal element wires 440 each having a circular cross section are twisted (10 element wires in this embodiment). Further, since the coil body 424 is heat-treated by the known method after the two or more metal element wires 440 are twisted, the residual stress of the coil body 424 is removed.

The same material as the metal element wire 40 in the first embodiment can be used for a metal material which constitutes the metal element wire 440 of the coil body 424.

The outer resin layer 428 constitutes the outermost layer which forms the outer surface of the catheter body 411. In addition, it extends throughout the full length of the catheter body 411 in a state where the entire outer periphery of the coil body 424 is covered such that the outer peripheries of the metal element wires 440 which form the coil body 424 are not exposed outside from the outer surface of the catheter body 411.

Note that for a resin material for forming the outer resin layer 428, a similar material to the outer resin layer 28 in the first embodiment can be used.

Meanwhile, the front end tip 412 provided at the front end of the catheter body 411 has an elongated tubular overall shape, and comprises a lumen communicating with the lumen 416 of the catheter body 411, and has a plasticity well higher than that of the catheter body 411. In addition, the above front end tip 412 has a tapered outer periphery in which the diameter is gradually decreased toward a forefront part 48.

Note that for a resin material for forming the front end tip 412, a similar material to the front end tip 12 in the first embodiment may be used.

The inner periphery of the front end tip 412 is formed with the inner resin layer 426 extending from the front end of the catheter body 411, and the coil body 424 which continues from the front end of the catheter body 411 is wound and embedded around the outer periphery of the inner resin layer 426 in the inside of the front end tip 412.

Moreover, a reinforcement body 430 extending from the front end of the catheter body 411 is fixed to the base end part of the front end tip 412. Thereby, the detachment of the front end tip 412 from the catheter body 411 can be prevented.

Here, the reinforcement body 430 may be any as long as it can be fixed to the front end tip 412 and the coil body 424, but it is preferably formed in such a way to have a protrusion part 432 extending toward the outer periphery in order to effectively prevent the detachment of the front end tip 412 from the catheter body 411 in the axial direction of the catheter.

Note that the protrusion part 432 is formed at one position in this embodiment, but may be provided at two or more positions.

Finally, the an alternative of the third embodiment of the present invention will be described. The catheter according to this embodiment differs form the catheters according to the first and second embodiments in that the configuration of the reinforcement body is different. That is, the reinforcement body of the catheter according to this embodiment is integrally formed with the catheter body while the reinforcement bodies of the catheters according to the first and second embodiments are provided as separate bodies from the catheter bodies.

Note that since the catheter according this alternative has the same appearance as the catheter 10 according to the first embodiment, only the internal structure is described with reference to a drawing with an overall view omitted.

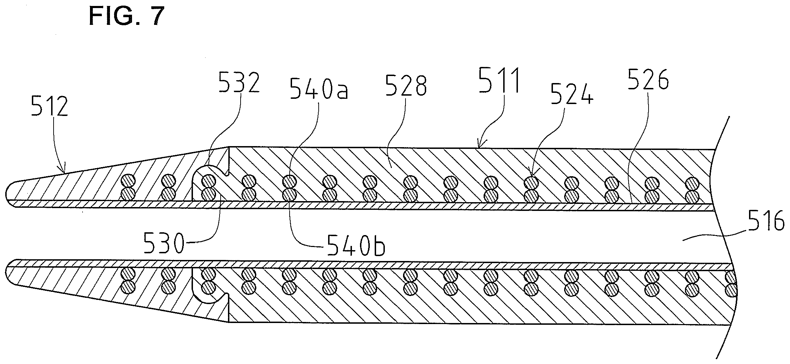

Fig. 7 shows a partial cross sectional view of the catheter according to the third alternative embodiment of the present invention. In Fig. 7, a catheter body 511 comprises a hollow elongated member having a lumen 516 into which a guide wire will be inserted. As shown in Fig. 7, the catheter body 511 has a braid body 524, an inner resin layer 526 covering the inner periphery of the braid body 524 and an outer resin layer 528 covering the outer periphery of the braid body 524.

The inner resin layer 526 has a tube-like shape extending throughout the full length at the innermost part of the catheter body 511. Moreover, the inner resin layer 526 axially protrudes from the front end of the catheter body 511 to the side of the front end. Note that for a resin material for forming the aforementioned inner resin layer 526, a similar material to the inner resin layer 26 in the first embodiment may be used.

The braid body 524 has a structure in which two metal element wires 540a and 540b are woven each other so as to form a mesh-like state. In addition, the braid body 524 having the aforementioned mesh-like structure is wound around the outer periphery of the inner resin layer 526 throughout the full length.

Further, as in the inner resin layer 526, the braid body 524 is wound around the outer periphery of the inner resin layer 526 which axially extends from the front end of the catheter body 511 to the side of the front end, and protruded from the front end of the catheter body 511.

The same material as the metal element wires 140a and 140b of the braid body 124 in the second embodiment may be used for a metal material which constitutes the metal element wires 540a and 540b of the braid body 524.

The outer resin layer 528 constitutes the outermost layer which forms the outer surface of the catheter body 511. In addition, it extends throughout the full length of the catheter body 511 in a state where the outer periphery of the braid body 524 is covered such that the outer peripheries of the metal element wires 540a and 540b which form the braid body 524 are not exposed outside from the outer surface of the catheter body 511.

Note that for a resin material for forming the outer resin layer 528, a similar material to the outer resin layer 28 in the first embodiment can be used.

Meanwhile, the front end tip 512 provided at the front end of the catheter body 511 has an elongated tubular overall shape, and comprises a lumen communicating with the lumen 516 of the catheter body 511, and has a plasticity well higher than that of the catheter body 511. In addition, the above front end tip 512 has a tapered outer periphery in which the diameter is gradually decreased toward a forefront part 48.

Note that for a resin material for forming the front end tip 512, a similar material to the front end tip 12 in the first embodiment may be used.

The inner periphery of the front end tip 512 is formed with the inner resin layer 526 extending from the front end of the catheter body 511, and the braid body 524 which continues from the front end of the catheter body 511 is wound and embedded around the outer periphery of the inner resin layer 526 in the inside of the front end tip 512.

Moreover, the reinforcement body 530 extending from the front end of the catheter body 511 is fixed to the base end part of the front end tip 512. Thereby, the detachment of the front end tip 512 from the catheter body 511 can be prevented.

Here, the reinforcement body 530 may be any as long as it can be fixed to the front end tip 512 and the braid body 524, but it is preferably formed in such a way to have a protrusion part 532 extending toward the outer periphery in order to effectively prevent the detachment of the front end tip 512 from the catheter body 511 in the axial direction of the catheter. This can further prevent the detachment of the front end tip 512 from the catheter body 511, which can, in turn, prevent the detachment of the front end tip 512 from the catheter body 511.

Note that the protrusion part 532 is formed at one position in this embodiment, but may be provided at two or more positions.

The embodiments of the present invention are described above in detail, but the present invention shall not be construed in any limitative manner, the scope of the invention being defined by the appended claims.

For example, in the embodiments described above, all the reinforcement bodies are each illustrated to have a larger diameter than the coil body or the braid body, but the reinforcement body does not necessarily have a lager diameter than the coil body or the braid body.

Nonetheless, in a case where the reinforcement body has a larger diameter than the coil body or the braid body, the detachment of the front end tip from the coil body or the braid body can further be prevented.

Further, in the aforementioned embodiments, the reinforcement body is described to comprise a resin harder than that constituting the front end tip, but the reinforcement body does not necessarily comprise a resin harder than that constituting the front end tip.

Nonetheless, in a case where the reinforcement body comprises a resin harder than that constituting the front end tip, the detachment of the front end tip from the coil body or the braid body can further be prevented.

Moreover, the coil body in the aforementioned embodiments comprises 10 metal element wires having the same diameter. However, the coil body, for example, may comprise 8 metal element wires having the same diameter and the rest 2 metal element wires having a different diameter or may comprise only a single metal element wire.

Nonetheless, two or more element wires are preferably used to form a coil body because the elongation of the coil body can be reduced in case that the front end tip is detached from the catheter body.

Moreover, for the coil body in the aforementioned embodiments, each metal element wire constituting the coil body is arranged in such a way to be spaced in the axial direction of the catheter body, showing a so-called sparsely wound sate. Nonetheless, in a case where the enhanced stiffness of a catheter is preferred, or the enhanced torque transmittability from the base end of a catheter to the front end is preferred, a so-called densely wound sate is preferred in which each metal element wires is arranged in such a way to make contact with each other.

Further, the braid body in the aforementioned embodiments is formed with two inter-woven metal element wires having the same diameter, but it may be formed with a braid body comprising metal element wires having different diameters. Further the braid body may be formed not only with two inter-woven metal element wires but also with more than two inter-woven metal element wires, and may be formed with clockwise metal element wires and counter-clockwise metal element wires, the numbers of the clockwise metal element wires and the counter-clockwise metal element wires being different.

Moreover, the element wires constituting the braid body in the aforementioned embodiments are metal element wires, but the braid body may be formed with woven resin element wires, or may be formed with a metal element wire and a resin element wire woven each other in combination. Furthermore, the number of element wires and the braiding manner of element wires to form a braid body may appropriately be altered.

Further, the metal element wire which constitutes the coil body and the braid body in the aforementioned embodiments has a circular cross section, but the coil body and the braid body may be formed with an element wire with an elliptical cross section or an element wire with a rectangular cross section.

Moreover, the protrusion part in the aforementioned embodiments is described to have a curved shape on the whole, but not limited to it. For example, the protrusion part may have a sharply pointed shape on the front end tip, or may enter into the front end tip in a wedge-like manner.

Furthermore, in the aforementioned embodiments, the coil body or the braid is arranged at the outer periphery of the inner resin layer in view of that the coil body and the braid can easily be formed. Nonetheless, the inner resin layer may be integrally formed with the outer resin layer, and then a coil body or a braid may be arranged in the inside of an additional resin layer. Further, in addition to the two-layered structure of an inner resin layer and an outer resin layer, even a configuration in which a coil body or a braid is arranged in a certain layer of a multilayered structure having three or more layers can be used for the present invention.

Moreover, a configuration in which a coil body alone or a braid alone is arranged in each catheter is described in the aforementioned embodiments, but even a configuration in which a coil body and a braid are both arranged in one catheter can be used for the present invention.

### DESCRIPTION OF SYMBOLS

10 Catheter
11, 111, 211, 311, 411, 511 Catheter body
12, 112, 212, 312, 412, 512 Front end tip
14 Connector
16, 116, 216, 316, 416, 516 Lumen
18 Front end part
20 Intermediate part
22 Base end part
24, 224, 424 Coil body
26, 126, 226, 326, 426, 526 Inner resin layer
28, 128, 228, 328, 428, 528 Outer resin layer
30, 130, 230, 330, 430, 530 Reinforcement body
40, 140a, 140b, 240, 340a, 340b, 440, 540a, 540b Metal element wire
48 Forefront Part
124, 324, 524 Braid body
132, 232, 332, 432, 532 Protrusion part

## Claims

1. A catheter (10) comprises:
a catheter body (111, 211, 311, 411, 511) having a lumen (116, 216, 316, 416, 516),
a resin front end tip (112, 212, 312, 412, 512) having a lumen communicating with the lumen (116, 216, 316, 416, 516) of the catheter body (111, 211, 311, 411, 511), and attached to a front end of the catheter body (111, 211, 311, 411, 511),
a coil body (224, 424) and/or a braid (124, 324, 524) arranged at the catheter body (111, 211, 311, 411, 511) and the front end tip (112, 212, 312, 412, 512) along the lumen (116, 216, 316, 416, 516), and
a reinforcement body (130, 230, 330, 430, 530) covering the coil body (224, 424) and/or the braid (124, 324, 524) and being provided at the front end tip (112, 212, 312, 412, 512),
**characterized by** that the reinforcement body (130, 230, 330, 430, 530) has a protrusion part (132, 232, 332, 432, 532) extending toward an outer periphery of the front end tip (112, 212, 312, 412, 512).

2. The catheter (10) according to claim 1, **characterized by** that the reinforcement body (230, 330, 430, 530) is arranged in such a way to span the catheter body (211, 311, 411, 511) and the front end tip (212, 312, 412, 512).

3. The catheter (10) according to claim 1 or 2, **characterized by** that the reinforcement body (430, 530) is integrally formed with the catheter body (411, 511).

## Patentansprüche

1. Katheter (10) mit:
einem Katheterkörper (111, 211, 311, 411, 511), der ein Lumen (116, 216, 316, 416, 516) aufweist,
einer vordere Spitze (112, 212, 312, 412, 512), die ein Lumen aufweist, das mit dem Lumen (116, 216, 316, 416, 516) des Katheterkörpers (111, 211, 311, 411, 511) kommunziert, und die an einem vorderen Ende des Katheterkörpers (111, 211, 311, 411, 511) angebracht ist,
einem Wicklungskörper (224, 424) und/oder Geflecht (124, 324, 524), der oder das am Katheterkörper (111, 211, 311, 411, 511) und an der vorderen Spitze (112, 212, 312, 412, 512) entlang des Lumens (116, 216, 316, 416, 516) angeordnet ist, und
einem Versteifungskörper (130, 230, 330, 430, 530), der den Wicklungskörper (224, 424) und/oder das Geflecht (124, 324, 524) umhüllt und an der vorderen Spitze (112, 212, 312, 412, 512) vorgesehen ist, **dadurch gekennzeichnet, dass**
der Versteifungskörper (130, 230, 330, 430, 530) einen Vorsprung (132, 232, 332, 432, 532) aufweist, der sich in Richtung des Außenumfangs der vorderen Spitze (112, 212, 312, 412, 512) erstreckt.

2. Katheter (10) nach Anspruch 1, **dadurch gekennzeichnet, dass** der Versteifungskörper (230, 330, 430, 530) in der Weise angeordnet ist, dass er den Katheterkörper (211, 311, 411, 511) und die vordere Spitze (212, 312, 412, 512) überspannt.

3. Katheter (10) nach Anspruch 1 oder 2, **dadurch gekennzeichnet, dass** der Versteifungskörper (430, 530) mit dem Katheterkörper (411, 511) einstückig ausgebildet ist.

## Revendications

1. Cathéter (10) comprenant :
un corps de cathéter (111, 211, 311, 411, 511) ayant une lumière (116, 216, 316, 416, 516),
une pointe d'extrémité avant en résine (112, 212, 312, 412, 512) ayant une lumière communiquant avec la lumière (116, 216, 316, 416, 516) du corps de cathéter (111, 211, 311, 411, 511) et fixée sur une extrémité avant du corps de cathéter (111, 211, 311, 411, 511),
un corps de bobine (224, 424) et/ou une tresse (124, 324, 524) agencé(s) au niveau du corps de cathéter (111, 211, 311, 411, 511) et la pointe d'extrémité avant (112, 212, 312, 412, 512) le long de la lumière (116, 216, 316, 416, 516), et
un corps de renforcement (130, 230, 330, 430, 530) recouvrant le corps de bobine (224, 424) et/ou la tresse (124, 324, 524) et étant prévu au niveau de la pointe d'extrémité avant (112, 212, 312, 412, 512),
**caractérisé en ce que** le corps de renforcement (130, 230, 330, 430, 530) a une partie de saillie (132, 232, 332, 432, 532) s'étendant vers une périphérie externe de la pointe d'extrémité avant (112, 212, 312, 412, 512).

2. Cathéter (10) selon la revendication 1, **caractérisé en ce que** le corps de renforcement (230, 330, 430, 530) est agencé afin de couvrir le corps de cathéter (211, 311, 411, 511) et la pointe d'extrémité avant (212, 312, 412, 512).

3. Cathéter (10) selon la revendication 1 ou 2, **caractérisé en ce que** le corps de renforcement (430, 530) est formé de manière solidaire avec le corps de cathéter (411, 511).
